# EUROPÄISCHE PATENTSCHRIFT

(11) **EP 2 440 301 B1**
(45) Veröffentlichungstag und Bekanntmachung des Hinweises auf die Patenterteilung: **06.11.2013**
(21) Anmeldenummer: 10715508.7
(22) Anmeldetag: 23.04.2010
(51) Int. Cl.: B01D 15/38, A23J 1/00, A23J 1/14, A23L 1/211, A23L 2/72, A61K 36/00

(54) **VERFAHREN ZUR GEWINNUNG SEKUNDÄRER PFLANZENINHALTSSTOFFE**
METHOD FOR OBTAINING SECONDARY PLANT CONTENTS
PROCÉDÉ D'EXTRACTION DE SUBSTANCES VÉGÉTALES SECONDAIRES

(30) Priorität: 09.06.2009 DE 102009024410
(43) Veröffentlichungstag der Anmeldung: 18.04.2012
(73) Patentinhaber: Sartorius Stedim Biotech GmbH, 37079 Göttingen (DE)
(72) Erfinder: HÖRL, Hans-Heinrich, 37120 Bovenden (DE); DEMMER, Wolfgang, 37077 Göttingen (DE); FABER, René, 37077 Göttingen (DE)
(86) Internationale Anmeldenummer: PCT/EP2010/002497
(87) Internationale Veröffentlichungsnummer: WO 2010/142364

(56) Entgegenhaltungen:
- EP-A1- 0 806 474
- WO-A1-2008/097154
- WO-A1-2008/136741
- WO-A2-00/45769
- US-A- 5 141 611
- US-A- 5 886 155
- BORNEMAN Z ET AL: "Selective removal of polyphenols and brown colour in apple juices using PES/PVP membranes in a single-ultrafiltration process" JOURNAL OF MEMBRANE SCIENCE, ELSEVIER SCIENTIFIC PUBL.COMPANY. AMSTERDAM, NL LNKD- DOI:10.1016/S0376-7388(97)00105-1, Bd. 134, Nr. 2, 29. Oktober 1997 (1997-10-29), Seiten 191-197, XP004093667 ISSN: 0376-7388

## Beschreibung

Die vorliegende Erfindung betrifft ein Verfahren zum Isolieren von phenolischen sekundären Pflanzeninhaltsstoffen aus pflanzlichem Material, sowie einen durch dieses Verfahren erhältlichen Nahrungsmittelzusatz.

Pflanzliche Inhaltsstoffe, insbesondere die sogenannten sekundären Pflanzeninhaltsstoffe, die keinen größeren kalorischen Wert besitzen, geraten immer mehr in die Aufmerksamkeit der Wissenschaft und der Lebensmitteltechnologie, wo sie unter dem Stichwort "Functional Food" als Nahrungsergänzungsmittel bzw. Nahrungsmittelzusätze von Interesse sind.

Von besonderer Bedeutung sind hierbei niedermolekulare Verbindungen, die antioxidative Eigenschaften aufweisen und damit die ubiquitär entstehenden, sogenannten "reactive oxygen species", reaktive Sauerstoff-enthaltende Moleküle, wie zum Beispiel Hydroxyl-Radikale OH-, das Superoxidanion O₂⁻, Wasserstoffperoxid H₂O₂, Singulett-Sauerstoff, der mit Stickstoffmonoxid NO zu Peroxynitrit ONO₂ reagieren kann, sowie Hypobromit und Hypochlorit inaktivieren können. Im Zusammenhang mit ihrer antioxidativen Wirkung werden weitere positive Effekte dieser niedermolekularen Verbindungen, wie der Schutz endothelialer Zellen, die Unterdrückung von Tumorwachstum und der Schutz des Herz-Kreislaufsystems, intensiv diskutiert. Die kardioprotektive Wirkung zum Beispiel, die mit regelmäßigem Rotweinkonsum einhergeht, wird hierbei unter anderem durch mit OH-Gruppen substituierte Diphenole wie beispielsweise Resveratrol verursacht. Bei Resveratrol handelt es sich um einen Stoff, der seinerseits mutmaßlich als Antioxidans die Oxidationsempfindlichkeit des Lipoproteins geringer Dichte (low density lipoprotein, LDL) im Blut senkt, die Aggregation der Blutplättchen hemmt und durch Inhibition der Squalenmonooxygenase die endogene Cholesterolbiosynthese hemmt.

Die technische Gewinnung bzw. Isolierung sekundärer Pflanzeninhaltsstoffe stellt besondere Herausforderungen an deren Aufarbeitung, da die Stabilität vieler isolierter Substanzen oberhalb eines bestimmten pH-Werts rasch abnimmt. Da darüber hinaus viele Pflanzenextrakte einen hohen Gehalt an Fruchtsäuren und Ionen aufweisen, wird eine Auftrennung über konventionelle Ionenaustauschchromatographie erschwert oder sogar unmöglich gemacht. Im Stand der Technik bekannte Verfahren erfordern zudem oft hohe Volumina an einzusetzendem Pflanzenextrakt, benötigen toxikologisch bedenkliche oder ätzende Substanzen wie Methanol oder Essigsäure zur Elution der sekundären Pflanzeninhaltsstoffe, und sind oft sehr langwierig. Die eingeschränkte Lagerstabilität vieler sekundärer Pflanzeninhaltsstoffe verlangt dagegen nach raschen Prozeßabläufen, um die empfindlichen Substanzen möglichst schnell in ein stabilisierendes Milieu überführen zu können.

Im Stand der Technik bekannt ist beispielsweise ein Verfahren zur Anthocyan-Anreicherung aus Heidelbeersaftkonzentrat mittels einer zwei Meter langen, stark hydrophoben Amberlite^{®} XAD-Säule mit einem Durchmesser von 11 cm. Nachteilig bei diesem Verfahren sind allerdings die erforderlichen hohen Volumina an einzusetzendem Saftkonzentrat, die Elution mit toxikologisch bedenklichen bzw. ätzenden Substanzen, wie Methanol und Essigsäure, und der hohe Zeitbedarf für die Abtrennung der Anthocyane aus dem Saftkonzentrat.

WO 2008/136741 A1 offenbart ein Verfahren zur Entfernung von Polyphenolen aus Getränken, bei welchem die Getränke mit einer Polymermatrix behandelt werden, auf welcher Etherliganden, bevorzugt Polyetherliganden mit CC-Mehrfachbindungen, fixiert sind. Die Polymermatrix kann als partikuläres oder membranförmiges Adsorbens vorliegen.

WO 2008/097154 A1 offenbart ein Verfahren zur Trubstoffentfernung aus Getränken. Die verwendeten Polymermatrizen auf Basis von vernetzten Polysacchariden weisen eine Polyetherbeschichtung auf, welche durch Pfropfung von beispielsweise Polyethylenglycol oder Diethylen-glycol-vinylether herstellbar ist. Die Polyether-Funktionalisierung der Matrizen ermöglicht eine effiziente Entfernung von unerwünschten Polyphenolen aus Getränken, wobei die Polyphenole keine Zielprodukte des Verfahrens darstellen.

US 5,141,611 offenbart ein Verfahren zur Entfernung von Polyphenolen aus Getränken, wobei Polyamidmembranen oder -partikel mit einer Oberflächenmodifizierung auf Basis von Glutaraldehyd/Resorcinol oder auf Basis von Glutaraldehyd in Kombination mit Melamin, 1,6-Hexamethylendiamin oder verschiedenen Aminosäuren verwendet werden.

EP 0 806 474 A1 offenbart ein Verfahren, bei welchem Chromatographiegele auf Basis von Sepharose^{®}, welche kationenaustauschende Liganden (Sulfopropyl- oder Carboxymethylgruppen) aufweisen, zur Getränkeklärung oder -stabilisierung verwendet werden. Bei diesem Verfahren werden Polyphenole gleichzeitig mit begleitenden Proteinen als unerwünschte Kontaminanten aus Bier entfernt. Es wird desweiteren offenbart, wie die Kationenaustauscher durch Einwirkung von Wasser, Natronlauge oder Kochsalzlösung für die erneute Verwendung regeneriert werden können. Das aus EP 0 806 474 A1 bekannte Verfahren enthält keine Schritte, die eine Isolierung der Polyphenole als Zielsubstanzen erlauben, d. h. keine Schritte, die eine Abtrennung der Polyphenole von den begleitenden Proteinen oder anderen Kontaminanten aus der Bierherstellung erlauben.

US 5,886,155 offenbart ein Verfahren zur adsorptiven Abtrennung von Tanninen und Polyphenolen aus Proteingemischen pflanzlichen Ursprungs mittels hydrophober Interaktionschromatographie (HIC), wobei die Zielproteine von der HIC-Matrix eluiert werden, und die nachfolgende Reinigung der Zielproteine mittels eines zweiten Schrittes der hydrophoben Interaktionschromatographie.

WO 00/45769 A2 offenbart ein Verfahren zur Isolierung polyphenolischer Antioxidanzien aus einem purinhaltigen Pflanzenextrakt, wobei die Antioxidanzien selektiv an einer Matrix adsorbiert werden, die aus Polyvinylpolypyrrolidon, Chitosan oder Gemischen davon besteht, und wobei die Matrix z. B. als Chromatographieharz vorliegt.

Z. Borneman et al. offenbaren in Journal of Membrane Science 134 (1997), 191-197, ein Verfahren zur Entfernung von Polyphenolen und von einer mit der Anwesenheit dieser Polyphenole einhergehenden Braunfärbung aus Apfelsaft. Bezüglich der Entfernung dieser Polyphenole sind Polyethersulfonmembranen (PES), welche mit Polyvinylpyrrolidon modifiziert sind, Membranen aus regenerierter Cellulose, auf welcher keine Liganden fixiert sind, überlegen. Während des Verfahrens an der PES-Membran auftretende "Fouling"-Phänomene können durch Regeneration der Membran mit Natronlauge rückgängig gemacht werden.

Der vorliegenden Erfindung liegt daher die Aufgabe zugrunde, ein Verfahren zur Isolierung von sekundären Pflanzeninhaltsstoffen aus pflanzlichem Material bereitzustellen, welches auf einfache Weise, bei geringem Zeitaufwand und bei niedrigen pH-Werten durchgeführt werden kann. Dieses Verfahren soll außerdem die Verwendung von toxikologisch bedenklichen Lösungsmitteln, sowie die Verwendung großer Volumina von Lösungsmitteln zur Isolierung der sekundären Pflanzeninhaltsstoffe vermeiden.

Diese Aufgabe wird durch die in den Ansprüchen gekennzeichneten Ausführungsformen der vorliegenden Erfindung gelöst. Insbesondere wird erfindungsgemäß ein Verfahren zur Isolierung von sekundären Pflanzeninhaltsstoffen aus pflanzlichem Material bereitgestellt, das die obigen Anforderungen erfüllt. Dabei wurde überraschend gefunden, daß durch den Einsatz von mikroporösen Membranen, die gruppenspezifische Affinitätsliganden tragen, eine Extraktion von sekundären Pflanzeninhaltsstoffen aus pflanzlichem Material möglich ist.

Dementsprechend betrifft ein Gegenstand der vorliegenden Erfindung ein Verfahren zum Isolieren von phenolischen sekundären Pflanzeninhaltsstoffen aus pflanzlichem Material, umfassend die Schritte:
(a) das Bereitstellen eines Pflanzenextraktes aus pflanzlichem Material, welches phenolische sekundäre Pflanzeninhaltsstoffe enthält,
(b) das Kontaktieren des Pflanzenextraktes mit einer mikroporösen Membran, welche Affinitätsliganden für die phenolischen sekundären Pflanzeninhaltsstoffe aufweist, wodurch die phenolischen sekundären Pflanzeninhaltsstoffe an der Membran adsorbiert werden, und
(c) das Eluieren der phenolischen sekundären Pflanzeninhaltsstoffe von der Membran, wodurch eine die phenolischen sekundären Pflanzeninhaltsstoffe enthaltende Lösung erhalten wird,
wobei der Affinitätsligand der Membran aus der Gruppe, bestehend aus Boronaten und Metallchelaten, ausgewählt ist.

Der Begriff "Isolieren" umfaßt erfindungsgemäß auch das Erhalten einer die phenolischen sekundären Pflanzeninhaltsstoffe enthaltenden Lösung. Aus dieser Lösung können die phenolischen sekundären Pflanzeninhaltsstoffe gegebenenfalls weiter aufgereinigt werden und/oder die phenolischen sekundären Pflanzeninhaltsstoffe als solche durch Entfernen des Lösungsmittels erhalten werden.

Der Begriff "pflanzliches Material" umfaßt erfindungsgemäß jegliches pflanzliche Material, das phenolische sekundäre Pflanzeninhaltsstoffe enthält. In einer bevorzugten Ausführungsform ist das pflanzliche Material aus der Gruppe, bestehend aus Früchten, insbesondere Beeren, Gemüse, Hülsenfrüchten, Knollen, Zwiebeln, Rüben, Tee, Kakao, Kaffee, Holz, Blüten, Samen, Blättern und Zapfen von Nadelbäumen ausgewählt. In einer besonders bevorzugten Ausführungsform ist das pflanzliche Material Fruchthaut.

Der Begriff "sekundäre Pflanzeninhaltsstoffe" umfaßt erfindungsgemäß chemische Verbindungen, die von Pflanzen weder im Energiestoffwechsel noch im anabolen oder katabolen Stoffwechsel produziert werden, und die bevorzugt der Abwehr von Pathogenen und Herbivoren, dem Schutz vor Umwelteinflüssen, wie beispielsweise UV-Strahlung, oder dem Anlocken von Bestäubern und Samenverbreitern dienen.

Gemäß der vorliegenden Erfindung handelt es sich bei den sekundären Pflanzeninhaltsstoffen um phenolische Verbindungen. Im Rahmen der vorliegenden Erfindung werden unter phenolischen Verbindungen Phenole sowie Derivate von Phenolen verstanden. Solche Verbindungen können einen oder mehrere aromatische Ringe umfassen, wobei die aromatischen Ringe annelliert sein können oder durch substituierte Alkylgruppen miteinander verbrückt sein können und wobei die Derivate neben den phenolischen OH-Gruppen weitere OH-Gruppen aufweisen können. Außerdem können die phenolischen OH-Gruppen derivatisiert sein. Beispielsweise können die phenolischen OH-Gruppen und/oder die weiteren OH-Gruppen glykosyliert sein. Des Weiteren können die sekundären Pflanzeninhaltsstoffe durch Methylierung, Acetylierung oder Umwandlung in ihre Aldehyd- oder Säurefunktion modifiziert sein.

Die vorliegende Erfindung betrifft die Isolierung von jeglichen geeigneten phenolischen sekundären Pflanzeninhaltsstoffen. Vorzugsweise sind die sekundären Pflanzeninhaltsstoffen aus der Gruppe, bestehend aus Phenolen; Benzochinonen; Hydroxybenzoesäuren; Acetophenonen; Tyrosinderivaten; Phenylessigsäuren; Hydroxyzimtsäuren; Cumarinen; Isocumarinen; Chromonen; Naphthochinonen; Xanthonen; Stilbenen; Anthrachinonen; Flavonoiden, insbesondere Flavonen, Flavonolen, Flavanolen, Flavanonen, Flavanonolen, Anthocyanen, Proanthocyanen, Isoflavonoiden und Biflavonoiden; Lignanen, Neolignanen; Ligninen; Catecholmelaninen; Betalainen; und Chalkonen ausgewählt.

Ein bevorzugtes Phenol ist beispielsweise L-DOPA. Bevorzugte Hydroxyarylsäuren sind beispielsweise Hydroxybenzoesäuren, insbesondere Salicylsäure, 4-Hydroxybenzoesäure, Gentisinsäure, Protocatechusäure, Gallussäure, Vanillinsäure, Ellagsäure, Hexahydroxydiphensäure und deren Ester, insbesondere Tannine, sowie deren Dilactone. Bevorzugte Hydroxyzimtsäuren sind beispielsweise Cumarsäure, Ferulasäure, Kaffeesäure, Sinapinsäure, Rosmarinsäure, und deren Ester und Amide. Bevorzugte Cumarine sind beispielsweise Scopoletin, Herniarin, Aesculetin, Fraxetin, Cumarin und Umbelliferon. Ein bevorzugtes Stilben ist beispielsweise Resveratrol. Bevorzugte Flavonoide sind beispielsweise Flavone, Flavonole, Flavanole, Flavanone, Flavanonole, Proanthocyane und Anthocyane. Dabei sind bevorzugte Flavone beispielsweise Apigenin, Luteolin, Diosmetin, Chrysoeriol, Nobiletin, Apirgenin, Acacetin, Galangin, Chrysin, Tectochrysin, Scutellarein, Eupatorin, Genkwanin, Senensetin und deren Glykoside wie Hyperosid, Quercitrin und Hesperidin. Bevorzugte Flavonole sind beispielsweise Kämpferol, Quercetin, Myricetin und deren Arabinoside, Galactoside, Glucoside, Glycoside, Rhamnoside und Xyloside. Bevorzugte Flavanole sind beispielsweise Catechin, Epicatechin, Gallocatechin, Epigallocatechin, Theaflavin und deren Gallate. Bevorzugte Flavanone sind beispielsweise Isokuranetin, Naringenin, Hesperitin, Eriodictyol und deren Glycoside, Rutinosylderivate und Neohesperidosylderivate. Ein bevorzugtes Flavanonol ist beispielsweise Taxifolin. Bevorzugte Proanthocyane sind beispielsweise die Glycoside der Procyanidine und Prodelphinidine, insbesondere deren Gallate. Bevorzugte Anthocyane sind beispielsweise die Glycoside von Pelargonidin, Cyanidin, Päonidin, Delphinidin, Petunidin und Malvidin. Bevorzugte Isoflavonoide sind beispielsweise die Isoflavone, insbesondere die Soja-Isoflavone. Dabei sind bevorzugte Soja-Isoflavone beispielsweise Genistein, Daidzein, Glycetein und deren Glycoside. Bevorzugte Lignane sind beispielsweise die Flachs-Lignane, insbesondere Matairesinol und Secoisolariciresinol-Diglucoside.

Geeignete phenolische sekundäre Pflanzeninhaltsstoffe sind beispielsweise in K. Shetty, G. Paliyath, A. L. Pometto, R. E. Levin, "Functional Foods and Biotechnology", CRC Press LLC 2005, Taylor & Francis Group, S. 152-159, beschrieben.

Unter den genannten Verbindungen sind erfindungsgemäß insbesondere die Anthocyane, d.h. die Farbstoffe verschiedener Pflanzenteile, wie Fruchthäute, Beeren, oder Vakuolenbestandteile, insbesondere die Glycoside von Pelargonidin, Cyanidin, Päonidin, Delphinidin, Petunidin und Malvidin, bevorzugt. Ebenso sind die Proanthocyane auf der strukturellen Basis von Cathechin oder Epicathechin, auch in Form ihrer Gallate, besonders bevorzugt. Dem Fachmann sind die vielfältigen pflanzlichen Quellen für Anthocyane und andere sekundäre Pflanzeninhaltsstoffe bekannt. Ein Aufstellung findet sich beispielsweise in H.-D. Belitz und W. Grosch, "Lehrbuch der Lebensmittelchemie", 4. Auflage, H.-Springer-Verlag Berlin, Heidelberg, New York, 1992, S. 738-754, ISBN 3-540-55449-1.

Geeignete Verfahren zur Herstellung von Pflanzenextrakten aus pflanzlichem Material sind dem Fachmann bekannt, und umfassen beispielsweise die Homogenisierung von Pflanzenteilen in einem Dispergierer oder Homogenisator.

Der Pflanzenextrakt kann vor dem Kontaktieren mit der Membran beispielsweise durch eine Vorfiltration vorbehandelt werden. Eine solche Vorfiltration dient der Entfernung von Partikeln und Trubstoffen aus dem Pflanzenextrakt. In einer bevorzugten Ausführungsform der vorliegenden Erfindung wird der Pflanzenextrakt nach seiner Herstellung jedoch nicht weiter vorbehandelt. Insbesondere kann der Pflanzenextrakt ohne Vorfiltration mit der Membran kontaktiert werden. In diesem Fall enthält der Pflanzenextrakt Partikel und Trubstoffe und kann aufgrund dieser Bestandteile eine Trübung aufweisen. Die Größe der Partikel ist nicht beschränkt. In einer bevorzugten Ausführungsform der vorliegenden Erfindung weisen die in dem Pflanzenextrakt enthaltenen Partikel eine Größe von nicht mehr als 0,5 mm auf. Um dies zu erreichen, kann das erfindungsgemäße Verfahren in einer bevorzugten Ausführungsform nach Schritt (a) und vor Schritt (b) weiter den Schritt umfassen:
(a2) das Homogenisieren des Pflanzenextraktes derart, daß die im Pflanzenextrakt enthaltenen Partikel eine Größe von nicht mehr als 0,5 mm aufweisen.

Geeignete Verfahren zum Homogenisieren sind dem Fachmann bekannt. Ebenso sind dem Fachmann Verfahren zur Überprüfung der Teilchengröße bekannt, beispielsweise durch mikroskopische Kontrolle und computergestützte Bildauswertung.

Der Begriff "mikroporöse Membran" bezeichnet im Rahmen der vorliegenden Erfindung Membranen mit einer Porengröße von 0,1 bis 20 im, vorzugsweise 0,5 bis 15 im und mehr bevorzugt 1 bis 10 im. Die Bestimmung der Porengröße kann mit einem sogenannten "Capillary Flow Porometry Test" (Capillary Flow Porometer 6.0, CAPWIN Software System, Fa. Porous Materials Inc.) durchgeführt werden.
Die mikroporöse Membran kann in jeder Form vorliegen, die geeignet ist, daß die Oberflächen der Membran mit dem Pflanzenextrakt in Kontakt kommen. Beispielsweise kann die mikroporöse Membran in ein Membranadsorbermodul integriert sein. Geeignete Membranadsorbermodule sind beispielsweise aus der deutschen Patentanmeldung DE 102 36 664 A1 bekannt. Vorzugsweise ist ein solches Membranadsorbermodul partikelgängig, d.h. unempfindlich gegenüber Verstopfung durch partikel- und trubstoffhaltige Medien.

Die Integration der mikroporösen Membran in ein partikelgängiges Membranadsorbermodul ist insbesondere dann vorteilhaft, wenn ein partikelhaltiger Pflanzenextrakt verwendet wird. Die in dem Pflanzenextrakt enthaltenen Partikel beeinträchtigen die Adsorption der zu isolierenden phenolischen sekundären Pflanzeninhaltsstoffe an der Membran im Allgemeinen nicht. Eine Vorfiltration ist in diesem Fall nicht notwendig, was das erfindungsgemäße Verfahren vereinfacht.

Als mikroporöse Membran können alle Membranen verwendet werden, die zur Adsorption der phenolischen sekundären Pflanzeninhaltsstoffe befähigt sind. Dazu weisen die mikroporösen Membranen geeignete Affinitätsliganden auf.

Als Affinitätsliganden für phenolische sekundäre Pflanzeninhaltsstoffe können in dem erfindungsgemäßen Verfahren jegliche geeignete Liganden, die zur Bindung von OH-Gruppen befähigt sind, verwendet werden. Geeignete OH-Gruppen bindende Affinitätsliganden sind dem Fachmann bekannt. Gemäss der vorliegenden Erfindung sind die Affinitätsliganden Boronate oder Metallchelate. Unter den Metallchelaten sind besonders bevorzugt Metallkomplexe der Iminodiessigsäure, der N-Methyliminodiessigsäure, der N-(Hydroxymethyl)iminodiessigsäure, der N-(Hydroxyethyl)ethylendiamintetra-essigsäure und Kombinationen davon, jeweils bevorzugt als Komplexe mit Eisen-(II)-Kationen (Protein Purification, Second Edition, Principles, High Resolution Methods and Applications, J.-C. Janson, L. Ryden eds., Wiley-VCH 1998, ISBN 0-471-18626-0). Besonders bevorzugte Affinitätsliganden sind Aminophenylboronat oder Eisen-II-Iminodiessigsäurekomplexe. Geeignete Verfahren zur Derivatisierung von mikroporösen Membranen mit Affinitätsliganden sind dem Fachmann wohlbekannt, und sind beispielsweise in G. T. Hermanson, A. K. Mallia, P. K. Smith (Herausgeber), "Immobilized Affinity Ligand Techniques", Academic Press, San Diego, ISBN 0-12-342330-9, beschrieben.

In einer bevorzugten Ausführungsform der vorliegenden Erfindung wird beispielsweise Na-aminophenylboronat über die Aminogruppe als Imin/Schiffsche Base an die Aldehydgruppen der Ausgangsmembran gebunden. Die nach der Aufarbeitung der fertigen Membran vorliegenden beiden freien OH-Gruppen des auf der Membran als Ligand fixierten Aminophenylboronats erlauben die nachfolgende Interaktion mit cis-Diolen, zum Beispiel der Zuckerkomponente der Anthocyane oder Proanthocyane.

Eine zur Verwendung in dem erfindungsgemäßen Verfahren geeignete mikroporöse Membran ist beispielsweise eine Membran aus einer Cellulosehydrat-Matrix und Poren, die sich von einer Hauptoberfläche zur anderen Hauptoberfläche der Membran erstrecken, wobei die Membran auf ihren inneren und äußeren Oberflächen funktionelle Gruppen (Affinitätsliganden) zur adsorptiven Stofftrennung aufweist.

Als Ausgangsmaterial für eine solche mikroporöse Membran dient eine Celluloseester-Membran, die mit mindestens einer Lösung unter Bedingungen in Kontakt gebracht wird, die zum einen zu einer Quellung der Celluloseester-Matrix führen und zum anderen gleichzeitig, d.h. *in situ,* eine Hydrolyse (Verseifung) der Estergruppen zu Hydroxylgruppen bewirkt, wobei eine Cellulosehydrat-Membran entsteht.

Celluloseester-Membranen können aus Cellulosemonoacetat, Cellulosediacetat, Cellulosetriacetat, Cellulosepropionat, Cellulosebutyrat und Celluloseacetobutyrat oder anderen geeigneten Celluloseestern oder Cellulosenitrat, Methylcellulose oder Ethylcellulose, sowie Gemischen davon, aufgebaut sein.

Anschließend an die Verseifung wird die erhaltene Cellulosehydrat-Matrix bevorzugt durch Umsetzung der Hydroxylgruppen mit einem der mehreren, mindestens bifunktionellen Reagenz vernetzt. Danach werden in die vernetzte Matrix funktionelle Gruppen (Affinitätsliganden) zur Befähigung der adsorptiven Stofftrennung eingeführt.

In einem weiteren Schritt können funktionelle Gruppen, z.B. an die Hydroxylgruppen, der vernetzten Membran gebunden werden. Geeignete Methoden zur Bindung von funktionellen Gruppen sind dem Fachmann bekannt.

Bevorzugt werden funktionelle Gruppen über Epoxid-Gruppen oder Aldehyd-Gruppen an die Cellulosemembran gebunden. Die Einführung der Epoxid-Gruppen kann bereits in dem Vernetzungsschritt oder nachträglich stattfinden.

Besonders bevorzugt werden in dem erfindungsgemäßen Verfahren "Sartobind^{®}"-Membranen der Fa. Sartorius Stedim Biotech GmbH verwendet.

Der Schritt (b) des Kontaktierens des Pflanzenextraktes mit der mikroporösen Membran umfaßt alle Formen des Inkontaktbringens des Pflanzenextraktes mit der mikroporösen Membran. Dabei erfolgt das Inkontaktbringen in einer Weise, daß die phenolischen sekundären Pflanzeninhaltsstoffe auch mit den Affinitätsliganden der Membran in Kontakt treten, um an diese adsorbiert zu werden. Der Schritt (b) kann erfolgen, indem der Pflanzenextrakt beispielsweise tangential an mindestens einer Membranoberfläche entlanggeleitet wird. Es ist aber auch möglich, dass bei Verwendung einer Membran mit Poren, die sich von einer Hauptoberfläche zur anderen Hauptoberfläche der Membran erstrecken, der Pflanzenextrakt durch die Membran, welche bevorzugt konvektiv permeabel ist, geleitet wird.

Unter dem Begriff "Adsorption" werden im Rahmen der vorliegenden Erfindung sämtliche Möglichkeiten der reversiblen Bindung von phenolischen sekundären Pflanzeninhaltsstoffe an die Liganden verstanden. Diese reversible Bindung kann chemischer und/oder physikalischer Natur sein.

Gemäß der vorliegenden Erfindung erfolgt in Schritt (c) das Eluieren der phenolischen sekundären Inhaltsstoffe von der Membran. Auf diese Weise wird eine Lösung erhalten, welche die phenolischen sekundären Pflanzeninhaltsstoffe enthält. Unter dem Begriff "Elution" werden im Rahmen der vorliegenden Erfindung die Desorption und die damit einhergehenden Spülschritte zusammengefaßt. Die zur Elution verwendete Flüssigkeit ist das "Eluens".

Als Eluens kann in dem erfindungsgemäßen Verfahren jedes Lösungsmittel, das eine höhere Affinität zu den verwendeten Affinitätsliganden als der zu isolierende phenolische sekundäre Pflanzeninhaltsstoff aufweist, verwendet werden. In einer bevorzugten Ausführungsform des erfindungsgemäßen Verfahrens ist das Eluens aus der Gruppe, bestehend aus wäßrigen Zuckerlösungen und Citronensäurelösungen, ausgewählt. Als Zuckerlösungen können beispielsweise Lösungen von Glucose, Galaktose und Sorbit verwendet werden. Die Verwendung von solchen, toxikologisch unbedenklichen Lösungsmitteln hat den Vorteil, daß die erhaltene Lösung unmittelbar, d.h. ohne weitere Behandlung oder Aufbereitung, als gebrauchsfertiger Nahrungsmittelzusatz verwendet werden kann. So ist in einer weiteren bevorzugten Ausführungsform des erfindungsgemäßen Verfahrens das Lösungsmittel derart ausgewählt, daß die in Schritt (c) erhaltene, die phenolischen sekundären Pflanzeninhaltsstoffe enthaltende Lösung unmittelbar, d.h. ohne weitere Behandlung oder Aufbereitung, als gebrauchsfertiger Nahrungsmittelzusatz verwendet werden kann. Zu diesem Zweck geeignet sind insbesondere 1 M D-Sorbit-Lösungen oder 0,5 M Citronensäuremonohydrat-Lösungen.

Wird eine solche Zuckerlösung als Eluens und beispielsweise Phenylboronat als Affinitätsligand verwendet, so wechselwirkt der auf der Membran fixierte Phenylboronat-Ligand mit cis-Diolen, wie zum Beispiel Glucose, Galaktose oder Sorbit, die in der Elutionslösung enthalten sind. Dadurch werden die an den Affinitätsliganden gebundenen phenolischen sekundären Pflanzeninhaltsstoffe durch einen Überschuß an z.B. Sorbit vom Liganden verdrängt. Es resultiert eine z.B. sorbithaltige Lösung der phenolischen sekundären Pflanzeninhaltsstoffe als Eluat. Diese Lösung kann unmittelbar als gebrauchsfertiger Nahrungsmittelzusatz verwendet werden.

Wird beispielsweise eine Citronensäurelösung als Eluens und ein Eisen-II-iminodiessigsäurekomplex als Affinitätsligand verwendet, so werden nicht nur die phenolischen sekundären Pflanzeninhaltsstoffe eluiert, sondern auch teilweise die Metallionen aus dem auf der Membran fixierten Chelatbildner, beispielsweise Iminodiessigsäure, freigesetzt, die so als Eisen-II-citratkomplexe mit den phenolischen sekundären Pflanzeninhaltsstoffen in das Eluat übergehen. So wird ein gebrauchsfertiger Nahrungsmittelzusatz erhalten, der aus den phenolischen sekundären Pflanzeninhaltsstoffen und einem Mineralstoffzusatz (Eisen-II-citrat) besteht.

In einer weiteren bevorzugten Ausführungsform umfaßt das erfindungsgemäße Verfahren weiter nach Schritt (b) und vor Schritte (c) den Schritt:
(b2) das Entfernen von auf der Membran verbliebenen Pflanzenresten aus dem Pflanzenextrakt durch Waschen mit einem wäßrigen Medium.

Auf diese Weise kann der nachfolgende Schritt des Eluierens vereinfacht werden. Als wäßriges Medium für diesen Waschschritt sind solche bevorzugt, die zu keiner Elution der phenolischen sekundären Pflanzeninhaltsstoffe führen. Insbesondere wird Wasser als wäßriges Medium bevorzugt.

Die vorliegende Erfindung wird anhand der folgenden nicht-einschränkenden Beispiele näher erläutert.

### Beispiel 1: Herstellung eines Pflanzenextrakts

Im Folgenden ist die Herstellung eines Anthocyane enthaltenden Pflanzenextrakts beschrieben.

Der Pflanzenextrakt wurde aus kommerziell erhältlichen Pflaumen (*Prunus domestica*) (Unipack Fruits, Südafrika) hergestellt. Die Früchte hatten einen Durchmesser von 7 bis 8 cm, ein Frischgewicht von ca. 150 bis 200 g und zeichneten sich durch eine sehr dunkle, lila gefärbte Fruchthaut (Schale) aus. Von drei entsteinten Früchten wurde die Schale vom Fruchtkörper entfernt und mit einem "Ultra-Turrax^{®} T25" Dispergierer (Janke und Kunkel, Staufen im Breisgau) in 200 ml 10 mM HCl bei einer Leerlaufdrehzahl von 8000 Upm für 5 min homogenisiert. Es entstand eine trübe Suspension, wobei Partikel in einem Größenbereich von maximal 0,5 mm entstanden. Die Partikelgröße wurde mit einem Durchlichtmikroskop "Axiovert 40" (Zeiss) mit angeschlossener Kamera und der Bildauswertungssoftware "Axiovision" (Zeiss) bestimmt. Dabei wurde keine Größenverteilung bestimmt, sondern nur sichergestellt, daß die Partikel die Größe von 0,5 mm nicht überschreiten. Diese Suspension, im folgenden "Rohextrakt" genannt, wurde in den folgenden Beispielen verwendet.

### Beispiel2: Herstellung einer mikroporösen Membran mit Aminophenylboronat-Liganden:

Im Folgenden ist die Herstellung einer mikroporösen Membran mit Aminophenylboronat als Affinitätsligand beschrieben.

Verwendet wurde eine mit Polyestervlies verstärkte Celluloseacetat-Membran (CA-Membran) mit einem Porendurchmesser von ca. 3 µm, die einen Wasserdurchfluss von 730 ml/(min x bar x cm²) aufweist. Die Dicke der Membran betrug durchschnittlich 250 µm.

Diese CA-Membran wurde 30 min bei Raumtemperatur mit 0,6 M wäßriger Natronlauge-Lösung verseift und anschließend 3 x 10 min mit 0,5 M wäßriger Natronlauge-Lösung gespült. Die erhaltene Membran wurde 30 min bei Raumtemperatur mit wäßrigem, 15%igen 1,4-Butandioldiglycidylether in 0,5 M wäßriger Natronlauge-Lösung und 0,1%iger, wäßriger Natriumborhydridlösung behandelt (vernetzt). Danach wurde die feuchte Membran 20 h in einem abgeschlossenen Gefäß bei Raumtemperatur stehengelassen. Abschließend wurde 30 min mit fließendem Wasser gespült. Danach wurde die Membran durch 30minütige Behandlung mit einer 1%igen, wäßrigen Lösung von Natriumperiodat bei Raumtemperatur aktiviert und 15 min mit fließendem Wasser gespült.

In einer Pufferlösung, die 0,1 M Citronensäure enthielt und die mit Dinatriumhydrogenphosphat auf pH 5,6 eingestellt wurde, wurden 25 mg/ml Natrium-aminophenylboronat (FLUKA, Buchs, Schweiz; Best. Nr. 09199, Charge 374599/ 50399) gelöst. Zu 3 ml dieser Lösung wurden 60 cm² der oben beschriebenen Membran und 30 mg NaCNBH₃ (FLUKA, Buchs, Schweiz; Best. Nr. 71435, Charge 1358598/21208152) gegeben und bei Umgebungstemperatur 60 min auf einer Orbitalplattform "Certomat^{®} S" (Sartorius Stedim Biotech GmbH) bei 60 Upm geschüttelt. Danach wurden 50 mg NaBH₄ (FLUKA, Buchs, Schweiz; Best. Nr. 71321, Charge 1171186/32105122) zugegeben und für weitere 15 min geschüttelt. Dann wurde die Membran mit jeweils 300 ml Wasser dreimal für jeweils 10 min gewaschen. Als Ergebnis wurde eine Cellulosehydrat-Membran mit Aminophenylboronat als Affinitätsligand erhalten.

### Beispiel 3: Isolierung von Anthocyanen

Zu der in Beispiel 2 erhaltenen Membran wurden 7 ml des Rohextrakts aus Beispiel 1 gegeben und bei Umgebungstemperatur 20 min auf der obigen Orbitalplattform bei 60 Upm geschüttelt. Dabei färbte sich die Membran lila-rot. Die Membran wurde dann mit 2 x 300 ml 10 mM HCl in Wasser gewaschen und dann mit 50 ml einer Lösung von 1 M D-Sorbit (ROTH, Karlsruhe, Art. Nr. 6213.1, Charge 3499149) in 1 M HCl bei Umgebungstemperatur 20 min auf der Orbitalplattform bei 60 Upm geschüttelt. Es entstand ein rosarot gefärbter Überstand. Die Membran wurde dann mit 3 x 300 ml Wasser gewaschen und der Versuch mit derselben Membran insgesamt zweimal wiederholt (Versuch 1 bis Versuch 3 in Tabelle 1). Alle verwendeten Lösungen und Überstände wurden in einem Spektralphotometer bei einer Wellenlänge von 520 nm vermessen und so die Konzentration der Anthocyane bestimmt. Die Ergebnisse sind in der folgenden Tabelle 1 zusammengefaßt. Dazu wurden jeweils die Extinktionen der jeweiligen Fraktion in mg/l umgerechnet. Dabei erzeugt eine Lösung von 16 mg/l Anthocyangemisch bei pH 2,5 eine Extinktion von 0,45 bei der Wellenlänge 520 nm (L. Jurd, Food Sci. 29, 1964, S. 16-19).

**Tabelle 1: Ergebnisse der Beladung einer mit Aminophenylboronat umgesetzten, mikroporösen Membran mit einem Anthocyane enthaltenden Pflanzenextrakt.**

| | **Versuch 1** | **Versuch 2** | **Versuch 3** |
|---|---|---|---|
| Lösung zu Beginn⁺ [ig/total]⁺ | 785 | 2151 | 2151 |
| Lösung am Ende⁺ [ig/total]⁺ | 129 | 938 | 1386 |
| Eluat [ig/total]⁺ | 624 | 804 | 219 |
| Wiederfindung^{**} [%] | 95 | 66 | 29 |

| | | | |
|---|---|---|---|
| ⁺ entspricht 7 ml Rohextrakt * Konzentration der Anthocyane im Überstand über der Membran nach 20 min Schütteln der mit dem Rohextrakt behandelten Membran **= [c(Eluat) / (c(Lösung zu Beginn) - c(Lösung am Ende))] * 100% | | | |

Die Membran färbte sich im Verlauf der 3 Versuche intensiv rot an, da es zu einer irreversiblen Adsorption gefärbter Inhaltsstoffe kam. Gleichzeitig nahm die Wiederfindungsrate und Bindungskapazität für die Anthocyane im Verlauf von Versuch 1 bis 3 ab.

### Beispiel 4: Herstellung einer mikroporösen Membran mit Fe²⁺ beladener Iminodiessigsäure als Ligand

Im Folgenden ist die Herstellung einer mikroporösen Membran mit Fe²⁺ beladener Iminodiessigsäure als Affinitätsligand beschrieben.

Eine Membran-Ronde mit 57 mm Durchmesser und Metallchelat bildenden Gruppen des Typs Iminodiessigsäure (IDA) (Bezeichnung 19442, Charge 990136-3 R30 A, Sartorius Stedim Biotech GmbH) wurde in 20 ml einer Lösung von 0,5 Mol/l Eisen-II-chlorid in Wasser (E. Merck Darmstadt, Best. Nr. 1.03861.0250, Charge F1114661 246) gegeben und bei Umgebungstemperatur 10 min auf einer Orbitalplattform "Certomat^{®} S" (Sartorius Stedim Biotech GmbH) bei 60 Upm geschüttelt. Die Membran wurde durch die Aufnahme der Eisen-Ionen schwach hellgelb gefärbt. Danach wurde die Membran dreimal mit je 100 ml Wasser gewaschen. Dadurch wurde eine Membran mit durch Fe²⁺ komplexierter Iminodiessigsäure als Affinitätsligand erhalten.

### Beispiel 5: Isolierung von Anthocyanen

Die Membran aus Beispiel 4 wurde mit 7 ml Rohextrakt aus Beispiel 1 versetzt und bei Umgebungstemperatur 10 min auf der obigen Orbitalplattform bei 60 Upm geschüttelt. Dabei färbte sich die Membran tiefblau an. Danach wurde die Membran dreimal mit je 100 ml Wasser gewaschen. Die Membran wurde mit 29,5 ml einer Lösung von 0,5 Mol/l Citronensäuremonohydrat (Merck, Darmstadt) in 1 Mol/l HCl versetzt und bei Umgebungstemperatur 15 min auf der Orbitalplattform bei 60 Upm geschüttelt. Dabei entfärbte sich die Membran, gleichzeitig wurde der Überstand rosarot gefärbt.

Die rosarote Färbung des Überstands resultierte dabei aus der Freisetzung der Anthocyane, die an die Membran durch Chelatbildung mit dem Eisen-II-Iminodiessigsäurekomplex fixiert wurden.

Der Versuch wurde mit einer identischen Membran gemäß Beispiel 4 wiederholt. Die Ergebnisse der beiden Versuche sind in der folgenden Tabelle 2 zusammengefaßt. Dazu wurden jeweils die Extinktionen der jeweiligen Fraktion in mg/l umgerechnet. Dabei erzeugt eine Lösung von 16 mg/l Anthocyangemisch bei pH 2,5 eine Extinktion von 0,45 bei der Wellenlänge 520 nm.

**Tabelle 2: Ergebnisse der Beladung einer mit Iminodiessigsäure umgesetzten, Eisen-II-Ionen enthaltenden mikroporösen Membran mit einem Anthocyane enthaltenden Pflanzenextrakt**

| | **Versuch 1** | **Versuch 2** |
|---|---|---|
| Lösung zu Beginn [µg/total] | 1020 | 1450 |
| Lösung am Ende [µg/total] | 570 | 636 |
| Elution [µg/total] | 293 | 572 |
| Wiederfindung [%] | 65 | 70 |

Die Definitionen entsprechen den bei Tabelle 1 angegebenen.

### Beispiel 6: Herstellung eines partikelgängigen Membranadsorber-Moduls mit chelatbildenden Liganden

Analog zu der deutschen Patentanmeldung DE 102 36 664 A1 wurde ein partikelgängiges Membranadsorber-Modul hergestellt. Dazu wurde ein rechteckiges Stück der in Beispiel 4 beschriebenen Membran (Bezeichnung 19442, Charge 990136-3 R30 A, Sartorius Stedim Biotech GmbH) mit einer Breite von 8 cm und einer Länge von 100 cm zusammen mit einem Gewebe der gleichen Größe des Typs XNP 4410 (Convet Plastics bv, Genk, Belgien) auf einen Plastikstab mit 1 cm Durchmesser aufgewickelt und diese Packung in ein Plastikrohr mit 12 cm Länge und 47 mm Durchmesser zentriert so eingepaßt, daß die äußerste Membran-Wicklung der inneren Rohrwandung direkt und flüssigkeitsdicht anlag. Es resultierte daraus ein durch das Gewebe gebildeter, partikelgängiger Kanal, der auf beiden Seiten von der aufgewickelten Membran begrenzt wurde. Aus dem gleichen Gewebe wurden Ronden mit einem Durchmesser von 47 mm gestanzt. Je 8 dieser Ronden wurden auf die Packung auf der Anström- und Abströmseite des Wickels aufgelegt. Die Rohrenden wurden mit zentral durchbohrten und mit Schlaucholiven versehenen Gummistopfen geeigneter Größe verschlossen. Das Rohr wurde senkrecht in einen Halter eingesetzt und eine Schlauchpumpe mit einem Silikonschlauch an die Eingangsolive angeschlossen. An die Ablaufschlaucholive wurde ebenfalls ein geeigneter Silikonschlauch angeschlossen. Beide Schläuche wurden in ein Vorlagengefäß gesteckt. Das Totvolumen des gesamten Systems aus dem Adsorbermodul und den Schläuchen betrug 200 ml.

Das Vorlagengefäß wurde mit 500 ml Wasser befüllt und dieses für 5 min mit einer Flußrate von 300 ml/min durch das Adsorber-Modul zirkuliert. Das Wasser wurde verworfen, 300 ml frisches Wasser eingefüllt und eine Lösung von 1,5 g Eisen-II-chlorid (E. Merck, Darmstadt, Best. Nr. 1.03861.0250, Charge F1114661 246) in Wasser hinzugegeben. Die Lösung wurde wie oben über das Adsorber-Modul zirkuliert. Danach wurde das Modul dreimal mit je 500 ml Wasser gewaschen und die Waschlösung verworfen.

### Beispiel 7: Isolierung von Anthocaynen

Die Haut von zwei kommerziell erhältlichen Pflaumen (Prunus domestica) (Unipack Fruits, Südafrika) wurde in 150 ml 10 mM HCl mit einem Ultra-Turrax^{®}-Gerät (Janke und Kunkel) bei einer Leerlaufdrehzahl von 8000 Upm für 15 min homogenisiert. Danach wurden weitere 850 ml 10 mM HCl zugegeben, gründlich gemischt und in das Vorlagengefäß aus Beispiel 6 überführt. Es resultierte eine trübe, tiefrot gefärbte Suspension. Die Suspension wurde ohne weitere Vorbehandlung für 20 min über das Adsorber-Modul aus Beispiel 6 zirkuliert. Danach wurde 1 Liter einer Lösung von 10 mM HCl linear durch das Modul gefördert, bis der Ablauf klar und ungefärbt war. Der Ablauf wurde verworfen.

Anschließend wurden 0,3 l einer Lösung von 1 M Citronensäuremonohydrat in 1 M HCl (Eluens) in die Vorlage gegeben und diese Lösung für 20 min über das Adsorbermodul zirkuliert. Danach wurde die resultierende Elutionslösung bis auf das in der Membran zurückgehaltene Restvolumen aus dem Modul entfernt und gesammelt. Es resultierte eine tiefrosarot gefärbte Lösung. Das Modul wurde mit 2 l Wasser gewaschen.
Der Versuch wurde wiederholt, wobei nur 10 % des Rohextraktes (100 ml), bezogen auf den ersten Versuch, verwendet wurden.

Die Ergebnisse sind in der folgenden Tabelle 3 dargestellt. Es wurden jeweils die Extinktionen der jeweiligen Fraktion in mg/l umgerechnet. Dabei erzeugt eine Lösung von 16 mg/l Anthocyangemisch bei pH 2,5 eine Extinktion von 0,45 bei der Wellenlänge 520 nm.

**Tabelle 3: Ergebnisse der Beladung eines partikelgängigen Adsorber-Moduls, das eine mit Iminodiessigsäure umgesetzte, Eisen-II-Ionen enthaltende, mikroporöse Membran enthält, mit einem Anthocyane enthaltenden Pflaumenextrakt.**

| | **Versuch 1** | **Versuch 2** |
|---|---|---|
| Lösung zu Beginn [µg/total] | 177 | 14,1 |
| Lösung am Ende [µg/total] | 143,8 | 9,4 |
| Waschlösung [µg/total] | 9,6 | 1,7 |
| Eluat [µg/total] | 11 | 1,3 |
| Wiedefindung** [%] | 47 | 43 |

| | | |
|---|---|---|
| **= [c(Eluat) / (c(Lösung zu Beginn) - c(Lösung am Ende)) - c(Waschlösung)] * 100% Die übrigen Definitionen entsprechen den bei Tabelle 1 angegebenen. | | |

Die Ergebnisse zeigen, daß die hier verwendeten Membranen geeignet sind, phenolische sekundäre Pflanzeninhaltsstoffe aus pflanzlichem Material zu isolieren, ohne daß die eingesetzte Suspension geklärt werden muß.

## Patentansprüche

1. Verfahren zum Isolieren von phenolischen sekundären Pflanzeninhaltsstoffen aus pflanzlichem Material, umfassend die Schritte:
(a) das Bereitstellen eines Pflanzenextraktes aus pflanzlichem Material, welches phenolische sekundäre Pflanzeninhaltsstoffe enthält,
(b) das Kontaktieren des Pflanzenextraktes mit einer mikroporösen Membran, welche Affinitätsliganden für die phenolischen sekundären Pflanzeninhaltsstoffe aufweist, wodurch die phenolischen sekundären Pflanzeninhaltsstoffe an der Membran adsorbiert werden, und
(c) das Eluieren der phenolischen sekundären Pflanzeninhaltsstoffe von der Membran, wodurch eine die phenolischen sekundären Pflanzeninhaltsstoffe enthaltende Lösung erhalten wird,
wobei der Affinitätsligand der Membran aus der Gruppe, bestehend aus Boronaten und Metallchelaten, ausgewählt ist.

2. Verfahren nach Anspruch 1, wobei das pflanzliche Material aus der Gruppe, bestehend aus Früchten, insbesondere Beeren, Gemüse, Hülsenfrüchten, Knollen, Zwiebeln, Rüben, Tee, Kakao, Kaffee, Holz, Blüten, Samen, Blättern und Zapfen von Nadelbäumen, ausgewählt ist.

3. Verfahren nach Anspruch 1 oder 2, wobei die phenolischen sekundären Pflanzeninhaltsstoffe aus der Gruppe, bestehend aus Phenolen; Benzochinonen; Hydroxybenzoesäuren; Acetophenonen; Tyrosinderivaten; Phenylessigsäuren; Hydroxyzimtsäuren; Cumarinen; Isocumarinen; Chromonen; Naphthochinonen; Xanthonen; Stilbenen; Anthrachinonen; Flavonoiden, insbesondere Flavonen, Flavonolen, Flavanolen, Flavanonen, Flavanonolen, Anthocyanen, Proanthocyanen, Isoflavonoiden und Biflavonoiden; Lignanen, Neolignanen, Ligninen, Catecholmelaninen, Betalainen und Chalkonen, ausgewählt sind.

4. Verfahren nach Anspruch 3, wobei die phenolischen sekundären Pflanzeninhaltsstoffe aus der Gruppe, bestehend aus L-DOPA als einem Phenol; Hydroxybenzoesäuren, ausgewählt aus Salicylsäure, 4-Hydroxybenzoesäure, Gentisinsäure, Protocatechusäure, Gallussäure, Vanillinsäure, Ellagsäure und Hexahydroxydiphensäure, sowie deren Estern, insbesondere den Tanninen; Hydroxyzimtsäuren, ausgewählt aus Cumarsäure, Ferulasäure, Kaffeesäure, Sinapinsäure und Rosmarinsäure, sowie deren Estern und Amiden; Resveratrol als einem Stilben; Flavonolen, ausgewählt aus Kämpferol, Quercetin, Myricetin, sowie deren Arabinosiden, Galactosiden, Glucosiden, Glycosiden, Rhamnosiden und Xylosiden; Flavanolen, ausgewählt aus Catechin, Epicatechin, Gallocatechin, Epigallocatechin, Theaflavin, sowie deren Gallaten; Flavanonen, ausgewählt aus Isosakuranetin, Naringenin, Hesperitin, Eriodictyol, sowie deren Glycosiden, Rutinosylderivaten und Neohesperidosylderivaten; Anthocyanen, ausgewählt aus den Glycosiden von Pelargonidin, Cyanidin, Päonidin, Delphinidin, Petunidin und Malvidin; Proanthocyanen, ausgewählt aus den Glycosiden der Procyanidine und Prodelphinidine; Isoflavonen, ausgewählt aus Genistein, Daidzein, Glycetein, sowie deren Glycosiden; und Lignanen, ausgewählt aus Matairesinol und Secoisolariciresinol-Diglucosiden, ausgewählt sind.

5. Verfahren nach Anspruch 1, wobei der Affinitätsligand ein Metallchelat ist, ausgewählt aus der Gruppe, bestehend aus Metallkomplexen der Iminodiessigsäure, der N-Methyliminodiessigsäure, der N-(Hydroxymethyl)-iminodiessigsäure, der N-(Hydroxyethyl)ethylendiamintetraessigsäure und Kombinationen davon.

6. Verfahren nach Anspruch 1, wobei der Affinitätsligand der Membran Aminophenylboronat und/oder ein Eisen-II-Iminodiessigsäurekomplex ist.

7. Verfahren nach einem der Ansprüche 1 bis 6, wobei das zum Eluieren in Schritt (c) verwendete Lösungsmittel aus der Gruppe, bestehend aus wäßrigen Zuckerlösungen und Citronensäurelösungen, ausgewählt ist.

8. Verfahren nach einem der Ansprüche 1 bis 7, wobei das zum Eluieren in Schritt (c) verwendete Lösungsmittel derart gewählt wird, daß die in Schritt (c) erhaltene, die phenolischen sekundären Pflanzeninhaltsstoffe enthaltende Lösung unmittelbar als gebrauchsfertiger Nahrungsmittelzusatz verwendet werden kann.

9. Verfahren nach einem der Ansprüche 1 bis 8, nach Schritt (a) und vor Schritt (b) weiter den Schritt umfassend:
(a2) das Homogenisieren des Pflanzenextraktes derart, daß die im Pflanzenextrakt enthaltenen Partikel eine Größe von nicht mehr als 0,5 mm aufweisen.

10. Verfahren nach einem der Ansprüche 1 bis 9, nach Schritt (b) und vor Schritt (c) weiter den Schritt umfassend:
(b2) das Entfernen von auf der Membran verbliebenen Pflanzenresten aus dem Pflanzenextrakt durch Waschen mit einem wäßrigen Medium.

## Claims

1. Method of isolating phenolic secondary plant components from plant material, comprising the steps:
(a) providing a plant extract from plant material containing phenolic secondary plant components,
(b) bringing the plant extract into contact with a microporous membrane having affinity ligands for the phenolic secondary plant components, whereby the phenolic secondary plant components are adsorbed at the membrane, and
(c) eluting the phenolic secondary plant components from the membrane, whereby a solution containing the phenolic secondary plant components is obtained,
wherein the affinity ligand of the membrane is selected from the group consisting of boronates and metal chelates.

2. Method according to claim 1, wherein the plant material is selected from the group consisting of fruits, particularly berries, vegetables, legumes, tubers, bulbs, root tubers, tea, cocoa, coffee, wood, flowers, seeds, leaves and cones of conifers.

3. Method according to claim 1 or 2, wherein the phenolic secondary plant components are selected from the group consisting of phenols; benzoquinones; hydroxybenzoic acids; acetophenones; tyrosine derivatives; phenylacetic acids; hydroxycinnamic acids; coumarins; isocoumarins; chromones; naphthoquinones; xanthones; stilbenes; anthraquinones; flavonoids, particularly flavones, flavonols, flavanols, flavanones, flavanonols, anthocyans, proanthocyans, isoflavonoids and bioflavonoids; lignans, neolignans, lignins, catechol melanins, betalains and chalcones.

4. Method according to claim 3, wherein the phenolic secondary plant components are selected from the group consisting of L-DOPA as a phenol; hydroxybenzoic acids selected from salicylic acid, 4-hydroxybenzoic acid, gentisic acid, protocatechuic acid, gallic acid, vanillic acid, ellagic acid and hexahydroxydiphenic acid, as well as the esters thereof, particularly the tannins; hydroxycinnamic acids, selected from coumaric acid, ferulic acid, caffeic acid, sinapinic acid and rosmarinic acid, as well as the esters and amides thereof; resveratrol as a stilbene; flavonols, selected from camphor oil, quercetin, myricetin, as well as the arabinosides, galactosides, glucosides, glycosides, rhamnosides and xylosides; flavanols, selected from catechin, epicatechin, gallocatechin, epigallocatechin, theaflavin as well as the gallates thereof; flavanones, selected from isosakuranetin, naringenin, hesperitin, eriodictyol, as well as their glycosides, rutinosyl derivatives and neohesperidosyl derivatives; anthocyanins, selected from the glycosides of pelargonidin, cyanidin, peonidin, delphinidin, petunidin and malvadin; proanthocyanins, selected from the glycosides of procyanidin and prodelphinidin; isoflavones, selected from genistein, daidzein, glycetein, as well as the glycosides thereof; and lignans, selected from matairesinol and secoisolariciresinol diglucosides.

5. Method according to claim 1, wherein the affinity ligand is metal chelate, selected from the group consisting of metal complexes of iminodiacetic acid, N-methyliminodiacetic acid, N-(hydroxymethyl)-iminodiacetic acid, N-(hydroxyethyl)-ethylenediaminetetraacetic acid and combinations thereof.

6. Method according to claim 1, wherein the affinity ligand of the membrane is aminophenylboronate and/or an iron-II-iminodiacetic acid complex.

7. Method according to any one of claims 1 to 6, wherein the solvent used for elution in step (c) is selected from the group consist of aqueous sugar solutions and citric acid solutions.

8. Method according to any one of claims 1 to 7, wherein the solvent used for the elution in step (c) is selected in such a manner that the solvent, which is obtained in step (c) and contains the phenolic secondary plant components, can be used directly as a ready-for-use foodstuff additive.

9. Method according to any one of claims 1 to 8, comprising the further step after step (a) and before step (b) of:
(a2) homogenising the plant extract in such a manner that the particles contained in the plant extract have a size of not more than 0.5 millimetres.

10. Method according to any one of claims 1 to 9, comprising the further step after step (b) and before step (c) of:
(b2) removing plant residues, which remain on the membrane, of the plant extract by washing with an aqueous medium.

## Revendications

1. Procédé pour isoler des composants secondaires phénoliques de plantes à partir d'une substance végétale, comprenant les étapes :
(a) de préparation d'un extrait de plante à partir d'une substance végétale qui contient des composants secondaires phénoliques de plantes,
(b) de mise en contact de l'extrait de plante avec une membrane microporeuse qui présente des ligands d'affinité pour les composants secondaires phénoliques de plantes, ce par quoi les composants secondaires phénoliques de plantes sont adsorbés sur la membrane, et
(c) d'élution des composants secondaires phénoliques de plantes de la membrane, ce par quoi une solution contenant les composants secondaires phénoliques est obtenue,
le ligand d'affinité de la membrane étant choisi dans le groupe constitué par des boronates et des chélates de métaux.

2. Procédé selon la revendication 1, la substance végétale étant choisie dans le groupe constitué par des fruits, en particulier, des baies, des légumes, des légumes secs, des bulbes, des oignons, des betteraves, du thé, du cacao, du bois, des fleurs, des semences, des feuilles et des cônes de résineux.

3. Procédé selon les revendications 1 ou 2, les composants secondaires phénoliques de plantes étant choisis dans le groupe constitué par des phénols ; des benzoquinones ; des acides hydroxybenzoïques : des acétophénones ; des dérivés de tyrosine ; des acides phénylacétiques ; des acides hydroxy cinnamiques ; des coumarines ; des isocoumarines ; des chromones ; des naphtoquinones ; des xanthones, des stilbènes, des anthraquinones ; des flavonoïdes, en particulier des flavones, des flavolones, des flavanoles, des flavanonènes, des flavanolones, de l'anthocyanène, du proanthocyanène, des isoflavonoïdes et des biflavonoïdes ; des lignanes, des néolignanes, des lignines, des catécholmélanines, des bêtalaïnes et des chalcones.

4. Procédé selon la revendication 3 les composants secondaires phénoliques de plantes étant choisis dans le groupe constitué de L-DOPA sous forme de phénol ; des acides hydroxybenzoïques, choisis parmi l'acide salicylique, l'acide 4-hydroxybenzoïque, l'acide gentisique, l'acide protocatéchique, l'acide gallique, l'acide vanillique, l'acide ellagique et l'acide hexahydroxydiphénique, ainsi que leurs esters, en particulier les tannins ; des acides hydroxy cinnamiques, choisis parmi l'acide coumarique, l'acide férulique, l'acide caféique, l'acide sinapique et l'acide rosmarinique, ainsi que leurs esters et leurs amides ; le resveratrol en tant que stilbène ; des flavolones choisis parmi le camphrol, la quercétine, la myricétine, ainsi que leurs arabinosides, galactosides, glucosides, glycosides, rhamnosides et xylosides ; des flavanolènes choisis parmi la catéchine, l'épicatéchine, la gallocatéchine, l'épigallocatéchine, la théaflavine, ainsi que leurs gallates ; des flavanonènes choisis parmi l'isosacuranétine, la naringénine, l'hespéritine, l'ériodictyol, ainsi que leurs glycosides, leurs dérivés de rutinosyle et leurs dérivés de néohespéridosyle ; des anthocyanènes choisis parmi les glycosides de pélargonidine, de cyanidine, de paonidine, de delphinidine, de pétunidine et de malvidine ; des proanthocyanènes choisis parmi les glycosides de la procyanidine et de la prodelphinidine ; des isoflavones choisis parmi la génistéine, le daidzéine, la glycétéine, ainsi que leurs glycosides ; et des lignanènes choisis parmi le matairesinol et les diglucosides de sécoisolaricirésinol.

5. Procédé selon la revendication 1, le ligand d'affinité étant un chélate de métal, choisi dans le groupe constitué par les complexes métalliques de l'acide iminodiacétique, de l'acide N-méthyliminodiacétique, de l'acide n-(hydroxyméthyl)-iminodiacétique, de l'acide N-(hydrxyéthyl)éthylènediamine tétracétique et de leurs combinaisons.

6. Procédé selon la revendication 1, le ligand d'affinité de la membrane étant un aminophénylboronate et/ou un complexe de fer II et de l'acide iminodiacétique.

7. Procédé selon l'une des revendications de 1 à 6, où le solvant employé pour l'élution dans l'étape ⁰ est choisi dans le groupe constitué par des solutions de sucres aqueuses et des solutions d'acides citronique.

8. Procédé selon l'une des revendications de 1 à 7 où le solvant employé pour l'élution dans l'étape (c) est choisi de telle manière que le solvant contenant les composants secondaires phénoliques de plantes obtenus dans l'étape (c) peuvent être tout simplement employés comme des compléments alimentaires prêts à l'emploi.

9. Procédé selon l'une des revendications de 1 à 8 comprenant, en outre, après l'étape (a) et avant l'étape (b), l'étape :
(a2) d'homogénéisation de l'extrait de plante de telle sorte que les particules contenues dans l'extrait de plante présentent une taille inférieure à 0,5 mm.

10. Procédé selon l'une des revendications de 1 à 9, comprenant, en outre, après l'étape (b) et avant l'étape (c), l'étape :
(b2) d'enlèvement des restes végétaux de l'extrait de plante résiduels sur la membrane par un lavage avec un milieu aqueux.
